# EUROPEAN PATENT SPECIFICATION

(11) **EP 1 665 997 B1**
(45) Date of publication and mention of the grant of the patent: **28.12.2011**
(21) Application number: 04816151.7
(22) Date of filing: 17.09.2004
(51) Int. Cl.: A61B 18/20

(54) **DEVICE FOR PERFORMING AN OPTO-THERMO-MECHANICAL ACTION ON A BIOLOGICAL TISSUE**
VORRICHTUNG ZUR DURCHFÜHRUNG EINER OPTO-THERMO-MECHANISCHEN AKTION AN EINEM BIOLOGISCHEN GEWEBE
DISPOSITIF DE TRAITEMENT OPTIQUE, MECANIQUE ET THERMIQUE D'UN TISSU BIOLOGIQUE

(30) Priority: 18.09.2003 RU 2003128064
(43) Date of publication of application: 07.06.2006
(73) Proprietor: Arcuo Medical. Inc., Los Altos CA 94022 (US)
(72) Inventor: Sobol, Emil Naumovich, 127322 Moscow (RU); Bagratashvili, Viktor Nikolaevich, 119331 Moscow (RU)
(74) Representative: Hopfgarten, Nils
(86) International application number: PCT/RU2004/000454
(87) International publication number: WO 2005/025400

(56) References cited:
- EP-A- 1 279 374
- WO-A-02/32335
- WO-A-97/39690
- WO-A-98/55035
- WO-A2-01/22863
- DE-A1- 19 916 653
- RU-C1- 2 113 820
- RU-C1- 2 138 192
- RU-C2- 2 196 623
- SU-A1- 525 042
- US-A- 4 718 417
- US-A- 4 973 848
- US-A- 5 269 778
- US-A1- 2003 036 751
- US-B1- 6 542 767

## Description

### Field of the Invention

The invention relates to medicine, in particular to devices for medical treatment of biological tissues by locally modifying their structure and physical-chemical properties.

### Background of the Invention

A feedback-controlled laser treatment device is disclosed in US 2003/0036751 A1.

With deformation and degeneration of biological tissues associated are various diseases mostly treated by surgical methods, which methods are characterised by an high level of subsequent traumas, profuse bleeding, painfulness, necessity of narcosis and long inmating.

One known experimental work (E. Helidonis, E. Sobol, G. Kavalos et al., American Journal of Otolaryngology, 1993, Vol. 14, No. 6, pp. 410-412) for the first time described a method for changing a shape of the rabbit auricle cartilaginous tissue using a template and by irradiation emitted by CO₂-laser. According to said article, samples of the cartilaginous tissue 0.4-1 mm thick and having different initial deformation (curved and straight samples) are exposed out of an ear of a rabbit. Then by an external mechanical action the initial curved cartilaginous tissue is straightened manually, using forceps, and the straight configuration tissue samples are bent. Afterwards, using needles, these samples are secured on a wooden template and irradiated by CO₂-laser in the scanning mode. This technique provided a steady change of a shape of a preliminary exposed cartilaginous tissue intended for transplantation into a live organism. But due to the fact that the tissue is exposed out of an organism and a traumatising instrument is used, this technique is rather traumatic.

According to another known reference (E. Helidonis, E. Sobol, G. Velegrakis, J. Bizakis, Laser in Medical Science, 1994, Vol. 6, pp. 51-54), the preliminarily exposed samples of the cartilaginous tissue provided from human nasal septum and that of a rabbit were subjected to deformation using a template and CO₂-laser. This method provides stable results of changing a shape of the exposed cartilaginous tissue, when the tissue is preserved in physiologic saline. The method is applicable for reconstructive surgery, for which a cartilaginous tissue is exposed out of a patent's organism, then is treated mechanically and by a laser, with subsequent transplantation. Such operations cause traumas, are arduous and, furthermore, do not exclude a possibility of recurrence of the initial pathology. It should be appreciated that in said prior-art references cited are the results of the *in vitro* experiments, as the exposed cartilaginous tissue was subjected to the laser radiation outside an organism.

Known is a method for conducting a rhinological operation for modifying a human nasal septum cartilage shape (Patent RU No. 2114569, 07.09.93). The example described in this patent shows straightening of a curved human nasal septum using CO₂-laser.

According to said method: in the curvature area of a nasal septum - the mucous tunic is separated, the cartilage plate is straightened and held in the straightened state using a known clamp. This clamp usually comprises dual-branch, holding forceps having planar continuous branches, using which forceps the cartilage plate is captured, bent in the direction opposite to the pathological deformation, and is held during the entire time of irradiation. Then the cartilage is irradiated along the bending line by the scanning beam of CO₂-laser, rate of scanning being 0.03 cm/s. After said irradiation the clamp is removed and the modified shape of the nasal septum is inspected visually.

Though the above-mentioned clinical trials produced stable results, still suitability of said technique in real medical practice is rather conjectural. The technique lacks supervision over the cartilage irradiation course. The used radiation penetrates into the cartilage less than 50 mc deep, which results in inevitable overheating of the surface layer and destruction of perichondrium. According to the recited example of the clinical application of this method, mucous tunic and perichondrum are separated, which separation as such results in bleeding, a patient suffers pain, which subsequently may be conducive to atrophic processes.

Also known, for example, a method for modifying a shape of tracheal ring of a dog (Shapshay S.M., Pankratov M.M. et al., Ann. Otol. Rhinol. Laryngol., 1996, Vol. 105, pp. 176-181) using the laser radiation. This known method is used in cases of stenosis of larynx and trachea: to improve breathing, the contracted cartilage element of trachea is incised using an endoscope and CO₂-laser, and then, using the same endoscope, the deformed cartilage tissues is irradiated by Nd:YAG-laser, having wavelength of 1.44 mc, through the mucous tunic, along the internal surface of the trachea's contracted cartilage element.

This method is advantageous in terms of delivery of radiation and of performing the visual inspection of the treated zone, particularly when modified is a shape of cartilages of a difficult-access localisation. But this method is complicated technically and requires two successive laser-aided actions. Furthermore, transfer of the pathologically deformed area of a cartilage tissue into its normal position requires a significant external mechanical action. Such action is performed using a flexible endoscope that serves as a mechanical bougie. Said endoscope has to have a sufficient mechanical strength and rigidity. But in view of a limited mechanical strength of the endoscope, this method can be applied only for dilating the cartilage element having a relatively small radial deformation, about 1-2 mm at most.

Said method is capable of irradiating a cartilage only across the internal surface of the annular element, while the external side remains inaccessible for irradiation.

Known is the method/or treatment of a deformed cartilage tissue, and an instrument to carry out the same (Application WO 01/22863 A2, 05.04.2001, IPC A61B, Sobol, et al.), being the analogue most pertinent to the claimed technical solution.

The method is based on the laser-aided action, with simultaneous monitoring of the cartilage tissue parameters and varying of energy parameters of the laser-aided action.

Disadvantages of this method consist in that the positive outcome of change of a cartilaginous tissue shape is achieved in a narrow range of laser parameters, and a transgression beyond said range results in a damage to the tissue and recurrence of deformation; and the monitoring system employed in this method is based on measurement of the integral characteristic of a biological tissue, and dispenses with the spatial heterogeneity of said characteristics, which circumstance becomes the source of errors in selecting the moment when the laser-aided action should cease. An essential drawback of this method is also lack of supervision over characteristics of the biological tissue in the area contiguous to the area of the direct laser-aided action, which circumstance brings about premises that the surrounding tissues would be affected, and augments the risk of side effects. Besides, this method cannot be applied for treatment of damaged biological tissues, for example - articular cartilages and intervertebral disks.

Known is the method for treatment of the intervertebral disk diseases using the laser-aided ablation (vapourisation) of hernia and decompression of a disk (D. Choy DSJ, Case RB, Fielding W. Percutaneous laser nucleolysis of lumbar disc. New England Journal of Medicine, 1987, 317:771-772).

Drawbacks of this approach consist of inevitable overheating of the tissues adjacent to the ablation zone, and of undesirable action affecting the surrounding tissues, which action manifests itself in formation of a rough scar tissue; also there is an high probability of recurrence of a disease, for this method - similarly to the conventional surgical removal of the diskal hernia - fails to eliminate the fibrous ring defect, which defect, in many cases, is the basic cause of a disease.

Thus, presently there is no any efficient and safe approach to ensure the trauma-free treatment of the diseases associated with deformation of, and damage to biological tissues.

### Summary of the Invention

Said disadvantages and drawbacks are eliminated by a device as defined in the appended claim 1 for effecting the optical-thermal-mechanical (OTM) action on a biological tissue . Said device is configured to create the controlled spatial-temporal heterogeneities of temperature and heterogeneities of mechanical stresses in biological tissues by irradiating them by the optical irradiation, spatially and temporally modulated.

The spatial-temporal modulation (STM) of the optical radiation is a controlled, according to a predetermined rule, change in the spatial distribution of radiation power over time. STM includes the pulse-periodic nature of the laser radiation and the known rules for laser beam scanning, but differs from them by an arbitrarily predetermined spatial-temporal distribution of the optical radiation, and, as a consequence thereof, by a possibility to modify, according to a predetermined rule, the spatial and temporal characteristics of the optical heating and of the thermal stresses' fields, i.e. STM owns broader capabilities to effect the OTM-action upon biological tissue, in particular - capabilities to control gradients of temperature and mechanical stresses. As the investigations carried out in recent years ascertained, chondrocytes, fibroblasts and some other cells of biological tissue are responsive to the external mechanical stresses' fields, and in particular the productive and regenerative capacity of cells can be enhanced or diminished - depending on parameters of an external mechanical action. Presently there are no methods that would provide any controlled local thermal and mechanical action on cells in live organisms. The controllability requirement is needed to ensure effectiveness and predictability of the action's effects and results. The locality requirement is of importance due to the necessity to avoid an undesirable action on the surrounding tissues, to wit that the cells are to be safely acted upon.

It should be further noted that the claimed device provides formation of controllable and mutually matched spatial-temporal heterogeneities of temperature and thermo-mechanical stresses, as well as formation of acoustic waves in biological tissues.

A local thermal action to be effected on a biological tissue is required to perform a local irreversible change in micro-structure - "local melting of certain structural elements" of a biological tissue, which change leads to relaxation of mechanical stresses and creation of optimal heterogeneities of residual stresses in a tissue. As it occurs, the mechanical action on the tissues and, in particular, on biological cells thereof is exerted, the cells participate in the tissue regeneration process; and apart from that, the controlled thermal action increases the rate of all physical-chemical processes underlying the medical treatment. But overheating of the tissue results in its denaturation and destruction in the direct action zone, and also leads to undesirable consequences outside the zone (contrary to the locality and safety principles).

A long-term result of the medical treatment action on a biological tissue depends both on kinetics, a degree of completion of irreversible process, and also on distribution of residual stresses after the laser-aided action has ceased. As the residual stresses' field influences operation of the cells for regeneration of tissue, then for attaining the positive effect and ensuring safety of the procedure, the thermal and mechanical actions effected on a biological tissue should be matched.

The device is provided for effecting the optical-thermal-mechanical action on a biological tissue, providing an efficient and safe approach to trauma-free treatment of the diseases having causal nexus with deformation of, and damage to biological tissues, by way of creating the controlled residual stresses and the controlled spatial distribution of irreversible changes in the biological tissue structure.

Said result is to be achieved by applying the the OTM-action on a biological tissue, which method is based on implementation of the following steps:
on the basis of the pre-operative examination of a patient, the spatial distribution of physical-chemical and geometrical properties of the biological tissue in the area to be medically treated by the OTM-action is determined;
if required, the mechanical action on the biological tissue area to be medically treated is effected; and in particular, a predetermined shape is imparted to said area;
said biological tissue area is irradiated by the modulated and spatially formed - according to a predetermined rule - radiation within a range of optical lengths of the waves having predefined parameters; the irradiation being performed in conjunction with the simultaneous thermal and mechanical action effected on said area;
concurrently with said irradiation, in the biological tissue area performed is measurement of the spatial distribution of physical-chemical and geometric characteristics both in the direct optical action zone and outside thereof;
parameters of the radiation spatial formation and of the optical radiation modulation are matched mutually and with said characteristics of the biological tissue;
a change in said characteristics is determined with respect to measurements of said characteristics at the pre-operative action step;
the optical action parameters are controlled in the course of the irradiation depending on the continuously measured characteristics of the spatial distribution of physical-chemical and geometric characteristics both in the area of the direct action effected on the biological tissue and outside thereof;
once the required characteristics of the spatial distribution of the physical-chemical and geometric parameters have been obtained, irradiation of said biological tissue area is terminated; and as it occurs, parameters of the medical OTM-action on the biological tissue are pre-determined such that to ensure control of the residual mechanical stresses and to provide the process of the controlled irreversible modification of the biological tissue structure.

In so doing, radiation in the optical range of wavelengths is the laser radiation within the range of 0.1 to 11 µm.

The laser radiation can be of the pulsed or continuous nature.

Density of the laser radiation power is within the range of 1 to 1000 W/cm²

Duration of exposure of a biological tissue area to irradiation is selected within the period of 0.1 s- 30 min.

As this takes place, the spatial formation of the optical radiation, e.g: the laser radiation, is as follows:
formation of a pre-determined distribution of the radiation power density on the surface of, and in the bulk of the biological tissue area;
scanning of the laser beam along three axes according to a predetermined rule;
a combination of steps (a) and (b).

The optical radiation parameters, controlled in the course of the biological tissue area irradiation depending on the continuously measured characteristics of the spatial distribution of physical-chemical and geometric characteristics both of the area of the direct action effected on the biological tissue, and outside thereof are: a radiation wavelength, radiation power, power density and a spatial-temporal rule according to which said density varies, and also parameters of the modulation and spatial formation of laser radiation, for example - depth and frequency of modulation on the surface of, and in the bulk of the biological tissue, spatial distribution of radiation power.

A modulation depth according to the invention is 1-100%, a modulation frequency is within the bandwidth of 1-10⁹ Hz.

Measurements of the spatial distribution of physical-chemical and geometric characteristics, both in the direct laser-aided action zone and outside thereof, are done taking into account the spectral composition of the signal representing the biological tissue area's response to the modulated laser irradiation of said area.

The method further comprises the step of measurement of amplitude and phase of oscillations of the signal representing the biological tissue area's response to the modulated laser irradiation of said area.

Under these circumstances, a predetermined value of the laser radiation modulation frequency is selected such that it will match the resonance frequencies of mechanical vibrations in the area where the biological tissue is medically treated.

Further, if necessary, the biological tissue irradiation step is preceded by that of local application of brief pressures on biological tissue areas, for example on skin or mucous tunic that cover the medically treated tissue.

The device for effecting the medical treatment action on biological tissues comprises:
a source of optical radiation having: a unit for controlling the optical radiation power and temporal modulation, the source being optically coupled to a device for transferring the optical radiation and for forming the spatial distribution of the optical radiation power density on the surface of, and in the bulk of the biological tissue area; and also
a monitoring-diagnostic system that provides determination of the spatial distribution of the physical-chemical and geometric characteristics of the medically treated biological tissue area and of the area contiguous thereto;
the monitoring-diagnostic system being connected to: the optical radiation source, the unit for controlling the optical radiation power and temporal modulation, the device for transferring the optical radiation and forming the spatial distribution of the optical radiation power density on the surface of, and in the bulk of the biological tissue area, respectively.

In said device the optical radiation source comprises a laser radiation source.

The laser radiation source generates the laser radiation within the range of 0.1-11 µm.

The monitoring-diagnostic system includes at least one biological tissue condition sensor for measuring characteristics of the biological tissue area both in the medical treatment area and in immediate proximity thereto, which sensor is connected to a data processing unit that generates control signals for controlling the optical radiation parameters in the course of irradiation; and also a data visualisation and displaying device.

Here at least one biological tissue condition sensor in the monitoring-diagnostic system measures physical-chemical and geometric characteristics of the biological tissue area, such as temperature, water concentration in the biological tissue, mechanical stresses, light scattering characteristics, speed of sound, the optical-acoustic waves damping constant, geometric dimensions of biological tissue.

The unit for processing the signals supplied from the monitoring-diagnostic system basing on the signals, that arrive from at least one biological tissue condition sensor, delivers the control signals to the optical radiation source, to the unit for controlling the optical radiation power and temporal modulation, to the device for transferring the optical radiation and forming spatial distribution of the optical radiation power density on the surface of, and in the bulk of the biological tissue area, respectively.

The unit for controlling the optical radiation power and temporal modulation is implemented as an electro-optical modulator or acousto-optical modulator, or mechanical modulator.

Further, modulation of optical radiation is performed by varying the pump power, e.g. that of the laser radiation source.

The device for transferring the optical radiation and forming the spatial distribution of optical radiation power density on the surface of, and in the bulk of the biological tissue area is implemented as a forming optical system and electro-optical scanner, both being mutually optically coupled.

The device for transferring the optical radiation and forming the spatial distribution of the optical radiation power density on the surface of, and in the bulk of the biological tissue area is implemented as a forming optical system and a raster system, both being mutually optically coupled.

Said forming optical system is implemented as a segment of an optical fibre or a system of lenses and mirrors that delivers the laser radiation from the optical radiation source to the biological tissue area.

The inventive data visualisation and displaying device is implemented, for example, as an endoscope or display for displaying the biological tissue area, or as an optical coherent tomograph.

The data visualisation and displaying device performs measurement of geometric characteristics of the biological tissue area.

The feedback is executed by the monitoring-diagnostic system basing on the optical-thermal response of the biological tissue to the temporally-modulated laser radiation.

The feedback is also executed by the monitoring-diagnostic system basing on analysis of spectral composition of the biological tissue's signal responding to the laser's modulated action.

The feedback is executed by the monitoring-diagnostic system basing on analysis of amplitude and phase of the biological tissue's signal responding to the laser's modulated action.

The temporal rule of the laser radiation modulation - in particular: amplitude, depth, frequency and modulation mode - are determined by the monitoring-diagnostic system according to the pre-operative diagnostics data and updated in the course of the laser-aided action basing on a control signal from said monitoring-diagnostic system.

The rule for formation of the laser radiation spatial distribution is determined according to the pre-operative diagnostics data and updated in the course of the laser-aided action on the basis of a control signal from the monitoring-diagnostic system.

Parameters of the scanning process or of the laser radiation spatial distribution are determined according to the pre-operative diagnostics data and updated in the course of the laser-aided action on the basis of a control signal from the monitoring-diagnostic system.

In said device, matching of the rules for modulation and spatial formation of the laser radiation is done basing on the pre-operative diagnostics data, and said rules are updated in the course of the laser-aided action on the basis of the monitoring-diagnostic system's signal.

Besides, in said device, the feedback is executed on the basis of the biological tissue's optical-acoustic response to the modulated laser radiation generated in conformity with a pre-determined spatial distribution on the surface of, and in the bulk of the biological tissue.

The feedback is also executed on the basis of the optical-electrical response of the biological tissue to the modulated laser radiation generated according to a predetermined spatial distribution on the surface of, and in the bulk of the biological tissue.

The feedback is also executed on the basis of monitoring of changes of biological tissue's optical properties that take place when the tissue is exposed to the action effected by the laser radiation modulated and generated according to a pre-determined spatial distribution on the surface of, and in the bulk of biological tissue.

In the inventive device, at least one biological tissue condition sensor in the monitoring-diagnostic system can be positioned directly in the biological tissue area using a surgical instrument.

The device allows to:
lower the temperature whereat the medical treatment effect is achieved; broaden the range of acceptable modes of the effected action; broaden the field of safe employment of the method in medicine (in particular, for treatment of the spinal column diseases);
optimise (enhance) the medical optical-thermal-mechanical action effected on biological tissues, in particular by the (mechanical and acoustical) vibratory effects and resonances emerging therewith;
improve accuracy and reliability of operation of the feedback system;
avoid an undesirable action on the surrounding tissues, and diminish, or completely exclude, occurrence of complications and undesirable side effects.

The important peculiarity of the invention consists in that the use of the laser radiation modulation allows modify basically the operation of monitoring-diagnostic systems that will register the biological tissue's response exactly to the modulated action. Here the possibility to register the OTM-response, analyse the response spectral composition, its phase, and not only the signal amplitude, as it is the case, for example, according to Application WO 01/22863 A2, is provided.

### Brief Description of Drawings

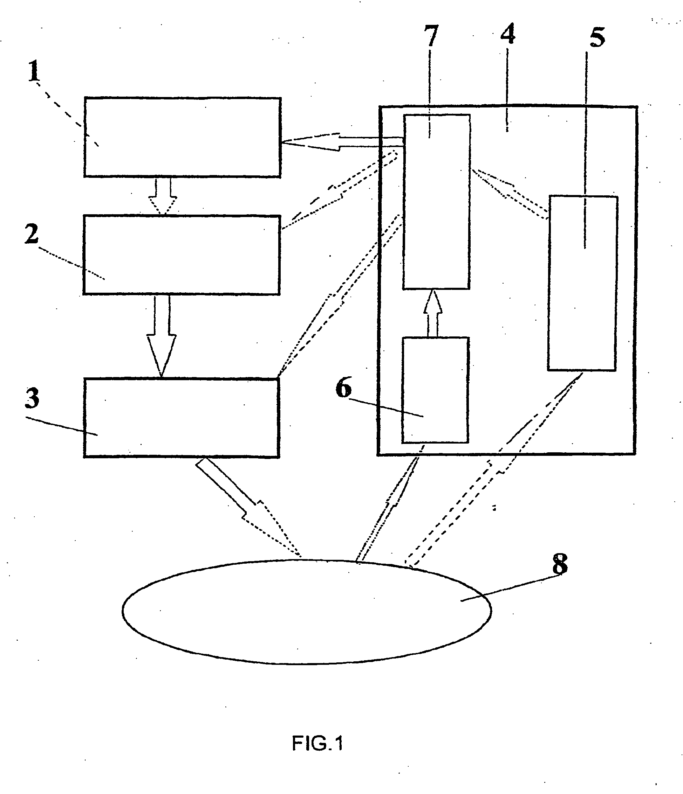

The invention is explained below by examples of embodiment thereof, with reference to the drawing, wherein
Fig. 1 shows a schematic diagram of a device for effecting the optical-thermal-mechanical action effected on said biological tissue.

### Best Modes for Embodying the Invention

The device for effecting a medical action on a biological tissue according to Fig. 1 consists: of a source 1 of optical radiation; a unit 2 for controlling the optical radiation power and temporal modulation; a device 3 for transferring optical radiation and forming the spatial distribution of a optical radiation power density on the surface of, and in the bulk of the biological tissue; a monitoring-diagnostic system 4; said system including: a data visualisation and displaying device 5, at least one biological tissue condition sensor 6, a data processing unit 7; the biological tissue area to be medically treated has reference numeral 8.

The optical radiation source 1 is a laser radiation source and can be implemented both as a pulse-periodic source and a continuous radiation source having the temporally-modulated output power. This can be, for example a pulse-periodic Nd:YAG laser having wavelength of 1.32 um, or a continuous fibre laser emitting the periodically-modulated radiation on wavelength 1.56 µm.

The unit 2 for controlling the optical radiation power and temporal modulation can be both an internal component of a laser excitation system, and an external unit not directly coupled to the laser. In the former case, the radiation is modulated by modulating a laser pump power, for example - by modulation of the power supply voltage. In the latter case, the radiation can be modulated, for example, using an electro-optical modulator, acousto-optical modulator (interrupter).

The device 3 for transferring the optical radiation and forming the spatial distribution of an optical radiation power density on the surface of, and in the bulk of the biological tissue can be implemented in two types. The first type uses periodic or aperiodic scanning of laser beam across a biological tissue along three axes. In this type, frequency and amplitude of scanning may vary so that to provide optimal conditions for the action to be effected on a tissue. As the scanner, an electro-optical scanner, for example, can be used.

In the second type: using an optical system (for example, a raster system), formed is a laser spot on the biological tissue surface having a pre-determined, in particular by the spatially-modulated radiation (e.g. periodically varying over space), distribution of the power density across the spot. The laser radiation is transferred from the radiation source (1) to the biological tissue using a forming optical system implemented as a system of lenses and mirrors, or as an optical fibre segment.

The monitoring-diagnostic system 4 consists of the data visualisation and displaying device 5, which device is implemented, for example, as an endoscope provided with a display or an optical coherent tomograph, at least one biological tissue condition sensor 6 and the data processing unit 7 that generates, basing on the signals from at least one biological tissue condition sensor, the controlling instructions for the optical radiation source 1, the unit 2 for controlling the optical radiation power and temporal modulation, and the device 3 for delivery of the optical radiation and forming the spatial distribution of the optical radiation power density.

The biological tissue condition sensor(s) 6 are the devices that register changes of physical-chemical properties of the biological tissue subjected to the OTM-action and, depending on a type of the medical action applied, type, localisation and dimensions of a medically treated biological tissue, they can be the customised sensors of temperature, amplitude, phase and frequency of acoustic signals, the mechanical stress sensors, the amplitude, phase and frequency sensors, as well as those of spatial distribution of scattered light, sensors of water concentration in the irradiated biological tissue.

The data processing unit (7) can be implemented as at least one computer circuit board, e.g. Intel Pentium-2 processor, audio card DC-XG Legacy Sound System, or a virtual multi-channel oscillograph, integrated with a computer and intended for processing the signals from the monitoring-diagnostic system's sensors (6) and for forming, in conformity with a specified algorithm, the control signals for the optical radiation source (1), for the unit (2) for controlling the optical radiation and power temporal modulation, and for the device (3) for transferring optical radiation and forming the power density spatial distribution - so that said units and devices will modify power of radiation, parameters of modulation and the spatial distribution of the optical radiation power density on the surface of, and in the bulk of the biological tissue, or will turn the laser off.

Radiation from the optical radiation source 1, basing on the pre-operative examination data, is modulated temporally using the unit 2 for controlling the optical radiation and temporal power modulation, and using the device 3 for transferring the optical radiation and forming the optical radiation power density spatial distribution, is generated and delivered to the irradiated biological tissue. At least one biological tissue condition sensor 6 is secured adjacently to, or in the direct contact with the irradiated tissue so that to ensure optimal obtainment of information of a biological tissue condition.

The method for effecting the OTM-action on a biological tissue is carried out as follows. An area of a given biological tissue to be medically treated is defined beforehand using, for example, the data visualisation and displaying device 5, or basing on data of the pre-operative tomography examination of a patient. Then the biological tissue condition sensor(s) 6 of the monitoring-diagnostic system 4 are disposed, and said monitoring-diagnostic system is turned on to determine the spatial distribution of physical-chemical and geometric properties of the biological tissue in the medical treatment area; for example the spatial distribution of mechanical stresses is measured by a stress-measuring micro-device, acoustic vibrations damping constant is measured during excitation of optical-acoustic waves by way of effecting a low-intensity modulated laser-aided action (power density being 0.01 - 0.5 W/cm²), at which intensity temperature changes in the laser-aided action zone would not exceed 1 K. Spatial distribution of temperature in the biological tissue is measured, for example, using a micro-thermocouple and a scanning infra-red imager. Geometric properties (shape and sizes) of the medically treated biological tissue area are determined using the data visualisation and displaying device 5, e.g. using an optical coherent tomograph. Spatial distribution of heterogeneity of the biological tissue structure is determined, for example, using an optical coherent tomograph.

Then the patient's pre-operative examination data are processed using the data processing device 7, which device, on the basis of a pre-determined algorithm, issues recommendations as to selection of initial parameters of irradiation by laser. In particular, a shape and sizes of the laser spot and a scanning rule are selected according to the geometric properties and spatial distribution of stresses in the medically treated biological tissue area. A pre-determined value of the optical radiation modulation frequency is selected, for example, such that it will match the resonance frequencies of mechanical vibrations in the medically treated biological tissue area. Afterwards the laser radiation parameters are determined, for example: wavelength - 1.5 µm; laser source power - 2 W, laser radiation spot shape - circle of 1 mm in diameter, for example; modulation frequency - 26 Hz, modulation depth - 80%, a rule for radiation scanning over space (along three axes) and over time.

If treated is a deformed cartilaginous tissue, then to the medically treated tissue is imparted, if necessary, a predefined shape by the mechanical action using a surgical instrument.

If required, a mechanical tool is also used for applying a local pressure onto areas of the biological tissue, for example - skin or mucous tunic that cover the medically treated biological tissue area. Application of such local pressure improves safety of the OTM-action. As said pressure is applied, water concentration decreases locally and, accordingly, radiation absorption coefficient locally diminishes in the near-surface layers of said biological tissue, which provides a shift of the temperature maximum into bulk of the medically treated biological tissue, and prevents overheating of, and damage to the surface layers of skin, mucous tunic and perichondrium.

The device for effecting the OTM-action on a biological tissue according to the invention is operated as follows.

Optical radiation, e.g. the laser radiation, from the radiation source 1 is modulated temporally using the unit 2 for controlling the optical radiation power and temporal modulation (for example, using an acousto-optical modulator), and using an optical forming system, for example, using an optical fibre, said radiation is supplied to the device 3 for formation of the spatial distribution of the optical radiation power density, being, for example, a micro-lens optical raster, disposed in vicinity of the surface (at distance of 5-10 mm) of the biological tissue irradiated area. Heating of the biological tissue by the laser radiation results in a change in the spatial distribution of its geometric and physical-chemical characteristics, such as field of temperatures, field of stresses and the laser radiation scattering diagram, which properties being continuously monitored by the data visualisation and displaying device 5, for example an optical coherent tomograph of IMALUX type; by the sensors 6, for example a scanning IR-radiometer or a stress-measuring micro-device based on a resistance stress gauge, or by an optical multi-channel analyser, for example - MORS-11.

Signals from the sensors 6 and the data visualisation and displaying device 5 are continuously output to the data processing unit 7, where the signals are processed and supplied to a video display for continuous visual monitoring to enable the visual supervision over characteristics of the irradiated tissue and to allow the manual control of the irradiation parameters. Concurrently, the data processing unit 7, basing on signals from the sensors 6 and data the visualisation and displaying device 5, generates, according to a specific algorithm, instructions for the optical radiation power and temporal modulation control device 2 and the device 3 for delivering the optical radiation and forming the spatial distribution so that to modify power, temporal modulation parameters and parameters of the spatial distribution of the optical radiation power density; and also generates the instruction to the optical source radiation 1 to turn off when the required characteristics of the irradiated biological tissue are attained, for example when temperature of nasal septum cartilage reaches 70°C.

Hereinafter, the method for effecting the OTM-action on biological tissues is set forth referring to the following particular examples thereof.

### Example 1

A male patient, 49, was referred to a clinic, with complaints to pains in lumbar part of spinal column, after one year as he had been operated for removal of intervertebral disk hernia. The pre-operative examination, including computerised tomography and discography, ascertained the presence of instability of spinal column and the presence of a defect in fibrous ring of the operated interverterbral disk.

For treating said pathology, first topology and dimensions of the defect were determined, and distribution of mechanical stresses in the fibrous ring area was determined using a strain-measuring micro-device inserted into the interverterbral disk through the needle 1.6 mm in diameter. As the laser radiation source, Er-glass fibre laser was selected and had wavelength of 1.56 µm, radiation power of 0.2-5 W, in said laser the radiation was modulated at frequencies within the bandwidth of 1-80 Hz and in depth of 50-100%. On the basis of the pre-operative diagnostics data, the following initial parameters of the laser radiation were selected: laser source power - 0.9 W, modulation frequency - 5 Hz, modulation depth - 80%. Local anesthesia (novocaine injection) was used. The radiation was delivered to the defect zone through a fibre-optic waveguide 600 µm in diameter, inserted into a metal needle 25 cm long and having outer diameter of 1.2 mm.

In the course of the operation, the following two sensors of a monitoring system were used:

An acoustic sensor for measuring the optical-acoustic response of a biological tissue to the modulated laser-radiation action; and a micro-thermocouple for temperature measurements. Both sensors were positioned on a second metal needle 25 cm long and 2 mm in diameter, which needle was inserted into the intervertebral disk at angle of 30 degrees with respect to the first needle and moved during radiation to a new position every 5 seconds, pitch being 0.5 mm. Position of both needles and the action area were visualised using an endoscopic system. Changes in the fibrous ring structure were registered using an optical coherent tomograph. Total time of effecting the action was 160 s. Temperature measurements showed that temperature increase in the fibrous ring near the tubus medullaris did not exceed 1.2°C, so that such low increase ensured safety of the OTM-treatment. Pains in the spinal column area were significantly soothed immediately after the procedure had been finished. The control examinations, conducted using a tomograph, discography and by a technique of measuring speed of propagation of acoustic waves after 3 and 9 months subsequent to the above-discussed treatment showed that the fibrous ring defect had been overgrown by the cartilaginous tissue. Thus, a correct selection of OTM-action modes to be effected by the modulated laser-aided action on the damaged intervertebral disk's fibrous ring enabled the process of the controlled irreversible modification of its structure and, as the result, provided the desired steady medical effect - the pain was relieved and spinal column became stable.

### Example 2

Female patient, 55, referred to the clinic with the request to eliminate some aesthetical disadvantages of her nose shape. The pre-operative examination using a data visualisation and displaying device, including an endoscope and optical coherent tomograph, showed a curvature of cartilage plates of nose wings, without any pathology in the osseous part of the nose.

As the laser radiation source, Nd:YAG solid-state pulsed-periodic laser was selected and had the following characteristics: wavelength - 1.32 µm, average radiation power - 0.3-5 W, pulse duration - 1 ms, pulse succession frequency: 10-700 Hz. The radiation spatial distribution unit was adapted to focus the radiation in the form of four circular spots having diameter of 0.4 - 3 mm, spaced at a distance 0.5 to 10 mm; the radiation was scanned along three axes at rate of 0.1-20 cm/s

As the feedback signal, the scattered radiation phase and the micro-thermocouple signal were used; the nose wings were additionally irradiated by a low-intensity light source provided by a diode laser having wavelength of 0.68 µm. Two symmetrically disposed nose wings' cartilages were given a new predetermined shape using a surgical instrument that provided a smooth curvature of the cartilages within the nose wings, without their surgical exposure. Two cartilage plates were irradiated alternately by the laser radiation pulses at frequency of 20 Hz through an optical fibre and optical raster system. Power of the laser radiation during first 12 s of irradiation was 2.5 W, then, after the micro-thermocouple's signal was received to indicate that temperature of the heated cartilage became steady at 52°C, the power was raised to 4.4 W. The laser was turned off after the monitoring-diagnostic system had outputted the signal as to the change of the light scattering signal phase by 180°C evidencing completion of the process of stress relaxation in the heated cartilage. For two different cartilage plates, the heating time by laser power of 4.4 W was 4.2 and 5.1 s, respectively, the same temperature of 68°C having been reached. The post-operative diagnostics using an optical coherent tomography and carried out immediately after the operation and 6 after months showed stability of the newly-imparted configuration of both nose wings, without visible damages to the mucous tunic and other adjacent tissues. Thus, selected modes of the OTM-action by the temporally modulated and spatially formed laser-aided action on the deformed nose wings' cartilage plates provided the process of the controlled irreversible modification and resulted in the desirable cosmetic effect - the deformed nose wings acquired the predetermined shape.

### Example 3

An adolescent, 13, was referred to the clinic with complaints to a difficult nasal breathing. The pre-operative examination with the use of an endoscopic visualisation system and optical coherent tomograph showed a curvature of the nasal septum cartilaginous part which emerged due to a trauma of the nose, without pathological alterations of the ossesous tissues.

As the laser radiation, selected was Er-glass fibre laser having wavelength of 1.56 µm, radiation power of 0.2-5 W, initial modulation of radiation of 365 Hz and depth of 30%.

A monitoring-diagnostic system based on an optical-acoustic sensor and strain-measuring micro-device was used. A laser source having a lowered power level of 0.1 W and spot 1 mm in diameter was employed and scanned linearly in the medically treated cartilaginous tissue area at frequency of 0.1 Hz and amplitude of 5 cm; in so doing, the spatial distribution of amplitude of the optical-acoustic signal was being measured, and basing on said distribution a data processing device (7 in Fig. 1) selected the initial laser power spatial distribution. Thus the laser spot on the mucous tunic surface, through which the cartilages were irradiated, was selected in the form of the strip 28 mm long and 0.3 mm wide and extending along the cartilage plate curvature line at distance of 5 mm from the cartilage growth zone; which strip ensured avoidance of overheating of the plate. The steps of straightening and fixation of a predetermined nasal septum shape, as well as the step of application of the mechanical pressure on the mucous tunic that covered the cartilaginous tissue in the medical treatment area, were carried out using a surgical instrument. The laser-aided heating was performed for 6 s, with the laser radiation power of 4.5 W. The laser was turned off after receipt of the stress-measuring micro-device signal indicating that the spatial heterogeneity of residual stresses in the nasal septum had been reached at level of 10%. Characteristic pitch of said heterogeneities was 300 µm, which value correlates with the characteristic distance between the active cells of the cartilaginous tissue - chondrocytes. In the course of the operation, performed with the use of the application anesthesia, the patient did not suffer any pain and left the clinic on his own after 30 minutes after the operation had been finished. The tomographical and rhinoscopic examinations, carried out immediately after the irradiation and also after 3 and 9 months, showed stability of the newly-imparted shape of the nasal septum cartilage and equality of gas flows via both nose airways. Optical coherent tomography did not find any damages in the mucous tunic adjacent to the nasal septum and in perichondrium. Thus, the selected modes of the OTM-action effected on the nasal deformed cartilage by the temporally-modulated and spatially-formed laser-aided action provided the controlled heterogeneity of residual mechanical stresses in the cartilaginous tissue, which resulted in the desired medical effect - straightening of the nasal septum and restoration of normal breathing. Besides, safety of restoration of the cartilage shape was ensured, because during the laser-aided OTM-action, the cartilage growth zone was not affected, which circumstance avoids any abnormalities of development and disproportions that may emerge as aftermath of the conventional, accompanied with traumas surgery.

The invention provides a novel device for effecting the controlled optical-thermal-mechanical action on the spatial heterogeneity of temperature of stresses and of structure of biological tissues. The claimed device for effecting the optical-thermal-mechanical action on a biological tissue can be suitably used in various fields of medicine, in particular in otolaryngology and cosmetology for correcting shapes of cartilaginous tissues, in ophthalmology for correcting cornea, in orthopedics and spinal surgery for treatment of diseases of joints and interverterbral disks

### Items on the Drawing

1 - laser radiation source
2 - unit for controlling parameters and modulating the radiation
3 - unit for transferring and forming the radiation spatial distribution
4 - monitoring-diagnostic system
5 - data visualization and displaying device
6 - biological tissue condition sensor(s)
7 - data processing unit
8 - area where the medical action is effected on a biological tissue

## Claims

1. A device for optical-thermal-mechanical treatment of biological tissue, comprising:
a mechanical tool for applying a local pressure onto areas of the biological tissue (8):
an optical radiation source (1) having an optical radiation power and a time modulation control unit, the device for treatment of biological tissue further comprising and optically coupled to a device (2, 3) for delivering optically modulated and spatially formed radiation, according to a predetermined rule;
a monitoring-diagnostic system (4) providing determination of the spatial distribution of the physical-chemical and geometric characteristics of the biological tissue area, and providing feedback on the basis of the measured spatial distribution of physical-chemical and geometric characteristics of the biological tissue area, said monitoring-diagnostic system being connected to the optical radiation source, the optical radiation power and time modulation control unit and the device for delivering the optical radiation and forming the spatial distribution of the optical radiation power density on the surface and in the bulk of the biological tissue area, respectively,
said monitoring-diagnostic system comprising at least one biological tissue condition sensor (6) to measure characteristics of the biological tissue area in the treatment region and in close proximity thereto;
wherein said monitoring-diagnostic system comprises a data processing unit (7) connected to said biological tissue condition sensor and configured to select a shape and size of the radiation spot and a scanning rule according to geometric properties and spatial distribution of stresses in the treated biological tissue area, and to select a predetermined value of the optical radiation modulation frequency in order to match the resonance frequencies of mechanical vibrations induced in the treated biological tissue area, and wherein said data processing unit is configured to generate, according to a specific algorithm, control signals to modify power, temporal modulation parameters and parameters of the spatial distribution of the optical radiation power density based on signals of said at least one biological tissue condition sensor (6).

2. The device as claimed in claim 1, wherein the optical radiation source (1) is a laser radiation source.

3. The device as claimed in claim 2, wherein the laser radiation source emits the laser radiation in a range from 0,1 to 11 micrometers.

4. The device as claimed in claim 1, wherein the monitoring-diagnostic system (4) comprises an information visualization and display device (5), and in that said data processing unit (7) is configured to generate control signals to control the optical radiation parameters in the irradiation process.

5. The device as claimed in claim 4, wherein the at least one biological tissue condition sensor (6) of the monitoring-diagnostic system is configured to measure biological tissue temperature and water concentration, mechanical stresses, light scattering characteristics, velocity of sound in the biological tissue, opto-acoustic waves damping factor, and geometric dimensions of the biological tissue.

6. The device as claimed in claim 4, wherein the data processing unit of the monitoring-diagnostic system is responsive to signals received from the at least one biological tissue condition sensor and is configured to provide control signals to the optical radiation source, to the optical radiation power and time modulation control unit, and to the device for delivering optical radiation and forming the spatial distribution of the optical radiation power density on the surface and in the bulk of the biological tissue, respectively.

7. The device as claimed in claim 1, wherein the optical radiation power and time modulation control unit is an electro-optical modulator, an acousto-optical modulator, or a mechanical modulator.

8. The device as claimed in claim 1, configured to modulate the optical radiation by modifying a pumping power of the laser radiation source.

9. The device as claimed in claim 1, wherein the device for delivering optical radiation and forming spatial distribution of optical radiation power density on the surface and in the bulk of the biological tissue includes a forming optical system and an electro-optical scanner, optically coupled to the forming optical system.

10. The device as claimed in claim 1, wherein the device for delivering optical radiation and forming spatial distribution of optical radiation power density on the surface and in the bulk of the biological tissue includes a forming optical system and a raster system, optically coupled to the forming optical system.

11. The device as claimed in any one of claims 9 or 10, wherein said forming optical system comprises a segment of optical fiber or a lens-and-mirror system adapted to deliver the laser radiation from the optical radiation source to the biological tissue area.

12. The device as claimed in claim 4, wherein the information visualization and display device (5) includes an endoscope and a display for displaying the biological tissue area, or an optical coherent tomography.

13. The device as claimed in claim 4, wherein the data visualization and display system (5) is configured to measure geometrical characteristics of the biological tissue area.

14. The device as claimed in claim 4, wherein feedback is provided by the monitoring-diagnostic system (4) on the basis of an optical-thermal response of the biological tissue to the time modulated and spatially formed laser radiation.

15. The device as claimed in claim 2, wherein feedback is provided by the monitoring-diagnostic system (4) on the basis of analysis of spectral distribution of the biological tissue response to the modulated laser radiation.

16. The device as claimed in claim 1, wherein feedback is provided by the monitoring-diagnostic system (4) on the basis of analysis of an amplitude and a phase of a biological tissue response to the modulated laser radiation.

17. The device as claimed in claim 2, configured to determine the time law of the laser radiation modulation, including modulation amplitude, modulation depth, modulation frequency and modulation mode, from pre-operative examination data and to update said law during the laser treatment responsive to a control signal from the monitoring-diagnostic system (4).

18. The device as claimed in claim 2, configured to determine the formation law of the laser radiation spatial distribution from pre-operative examination data and to update said law during the laser treatment responsive to a control signal from the monitoring-diagnostic system (4).

19. The device as claimed in claim 2, configured to determine the parameters of laser radiation scanning from pre-operative examination data and to update them during the laser treatment responsive to a control signal from the monitoring-diagnostic system (4).

20. The device as claimed in claim 2, configured to provide matching between the modulation law and the spatial formation law of the laser radiation on the basis of the pre-operative examination data and to update said matching during the laser treatment responsive to a control signal from the monitoring-diagnostic system.

21. The device as claimed in any one of claims 1 or 2, wherein feedback is provided on the basis of opto-electric signal from the biological tissue.

22. The device as claimed in claim 1, comprising a surgical instrument operable to position the at least one biological tissue condition sensor of the monitoring-diagnostic system in the biological tissue area.

23. The device as claimed in claim 22, configured for treatment of a cartilaginous tissue.

## Patentansprüche

1. Vorrichtung zur optisch-thermisch-mechanischen Behandlung von biologischem Gewebe, die umfasst:
ein mechanisches Werkzeug zum Ausüben eines räumlich begrenzten Drucks auf Bereiche des biologischen Gewebes (8);
eine Quelle (1) für optische Strahlung, die eine Steuereinheit für die Leistung optischen Strahlung und der zeitlichen Modulation hat, wobei die Vorrichtung zum von biologischem Gewebe ferner eine Vorrichtung (2, 3) enthält und mit optische gekoppelt ist, um optisch modulierte und räumlich gelformte Strahlung gemäß einer vorgegebenen Regel auszugeben:
ein Überwachungs-/Diagnose-System (4), die eine Bestimmung der räumlichen Verteilung der physikalischchemischen und geometrischen Eigenschaften des Bereichs biologischen Gewebes schafft und Rückkopplung anhand der gemessenen räumlichen Verteilung von physikalisch-chemischen und geometrischen Eigenschaften des Bereichs des biologischen Gewebes schafft, wobei das Überwachungs-/Diagnose-System mit der Quelle für optische Strahlung, mit Steuereinheit für die Leistung der optischen Strahlung und der zeitlichen Modulation und mit der Vorrichtung zum Liefern der optischen Strahlung und Bilden der räumlichen Verteilung der Leistungsdichte der optischen Strahlung auf der Oberfläche bzw. im Volumen des Bereichs des biologischen Gewebes verbunden ist,
wobei das Überwachungs-/Diagnosesystem wenigstens einen Sensor (6) für den Zustand des biologischen Gewebes umfasst, um Eigenschaften des Bereichs des biologischen Gewebes in dem Behandlungsbereich und in dessen enger Nachbarschaft zu messen;
wobei das Überwachungs-/Diagnose-System eine Datenverarbeitungseinheit (7) umfasst, die mit dem Sensor für den Zustand des biologischen Gewebes verbunden ist und konfiguriert ist, um eine Form und eine Größe des Strahlungsflecks und eine Abtastregel in Übereinstimmung mit geometrischen Eigenschaften und der räumlichen Verteilung von Beanspruchungen in dem behandelten Bereich des biologischen Gewebes zu wählen und um einen vorgegebenen Wert der Modulationsfrequenz der optischen Strahlung zu wählen, um die Resonanzfrequenzen mechanischer Vibrationen, die in dem behandelten Bereich des biologischen Gewebes induziert werden, zur Übereinstimmung zu bringen, und wobei die Datenverarbeitungseinheit konfiguriert ist, um in Übereinstimmung mit einem spezifischen Algorithmus Steuersignale zu erzeugen, um die Leistung, Zeitmodulationsparameter und Parameter der räumlichen Verteilung der Leistungsdichte der optischen Strahlung anhand von Signalen des wenigstens einen Sensors (6) für den Zustand des biologischen Gewebes zu modifizierten.

2. Vorrichtung nach Anspruch 1, wobei die Quelle (1) für optische Strahlung eine Laserstrahlungsquelle ist.

3. Vorrichtung nach Anspruch 2, wobei die Laserstrahlungsquelle die Laserstrahlung in einem Bereich von 0,1 bis 11 Mikrometer aussendet.

4. Vorrichtung nach Anspruch 1, wobei das Überwachungs-/Diagnose-System (4) eine Informationsvisualisierungs- und -anzeigevorrichtung (5) umfasst und dass die Datenverarbeitungseinheit (7) konfiguriert ist, um Steuersignale zu erzeugen, um die Parameter der optischen Strahlung in dem Bestrahlungsprozess zu steuern.

5. Vorrichtung nach Anspruch 4, wobei der wenigstens eine Sensor (6) für den Zustand des biologischen Gewebes des Überwachungs-/Diagnose-Systems konfiguriert ist, um die Temperatur und die Wasserkonzentration des biologischen Gewebes, mechanische Beanspruchungen, Lichtstreueigenschaften, Schallgeschwindigkeit in dem biologischen Gewebe, einen Dämpfungsfaktor für optischeakustische Wellen und geometrische Abmessungen des biologischen Gewebes zu messen.

6. Vorrichtung nach Anspruch 4, wobei die Datenverarbeitungseinheit des Überwachungs-/Diagnose-Systems auf Signals anspricht, die von dem wenigstens einen Sensor für den Zustand des biologischen Gewebes empfangen werden, und konfiguriert ist, um für die Quelle für optische Strahlung, für die Steuereinheit für die Leistung der optischen Strahlung und der zeitlichen Modulation und für die Vorrichtung zum Liefern von optischer Strahlung und zum Bilden der räumlichen Verteilung der Leistungsdichte der optischen Strahlung auf der Oberfläche bzw. im Volumen des biologischen Gewebes Steuersignale bereitzustellen.

7. Vorrichtung nach Anspruch 1, wobei die Steuereinheit für die Leistung der optischen Strahlung und der zeitlichen Modulation ein elektrooptischer Modulator, ein akusto-optischer Modulator oder ein mechanischer Modulator ist.

8. Vorrichtung nach Anspruch 1, die konfiguriert ist, um die optische Strahlung durch Modifizieren einer Pumpleistung der Laserstrahlungsquelle zu modulieren.

9. Vorrichtung nach Anspruch 1, wobei die Vorrichtung zum Liefern von optischer Strahlung und zum Bilden einer räumlichen Verteilung der Leistungsdichte der optischen Strahlung auf der Oberfläche und im Volumen des biologischen Gewebes ein optisches Formungssystem und einen elektrooptischen Scanner, der mit dem optischen Formungssystem optisch gekoppelt ist, enthält.

10. Vorrichtung nach Anspruch 1, wobei die Vorrichtung zum Liefern von optischer Strahlung und zum Bilden einer räumlichen Verteilung der Leistungsdichte der optischen Strahlung auf der Oberfläche und im Volumen des biologischen Gewebes ein optisches Formungssystem und ein Rastersystem, das mit dem optischen Formungssystem gekoppelt ist, enthält.

11. Vorrichtung nach einem der Ansprüche 9 oder 10, wobei das optische Formungssystem ein Segment einer Lichtleitfaser oder ein Linsen- und Spiegel-System umfasst, das dazu ausgelegt ist, die Laserstrahlung von der Quelle für optische Strahlung zu dem Bereich des biologischen Gewebes zu Liefern.

12. Vorrichtung nach Anspruch 4, wobei die Informationsvisualisierungs- und Anzeigevorrichtung (5) ein Endoskop und eine Anzeige zum Anzeigen des Bereichs des biologischen Gewebes oder eine optisch kohärente Tomographie umfasst.

13. Vorrichtung nach Anspruch 4, wobei die Informationsvisualisierungs- und -anzeigevorrichtung (5) konfiguriert ist, um geometrische Eigenschaften des Bereichs des biologischen Gewebes zu messen.

14. Vorrichtung Anspruch 4, wobei durch das Überwachungs-/Diagnose-System (4) anhand einer optischthermischen Antwort des biologischen Gewebes auf die zeitlich modulierte und räumlich geformte Laserstrahlung eine Rückkopplung geschaffen wird.

15. Vorrichtung nach Anspruch 2, wobei durch das Überwachungs-/Diagnose-System (4) anhand der Analyse der Spektralverteilung der Antwort des biologischen Gewebes auf die modulierte Laserstrahlung eine Rückkopplung geschaffen wird.

16. Vorrichtung nach Anspruch 1, wobei durch das Überwachungs-/Diagnose-System (4) anhand der Analyse einer Amplitude und einer Phase einer Antwort des biologischen Gewebes auf die modulierte Laserstrahlung eine Rückkopplung geschaffen wird.

17. Vorrichtung nach anspruch 2, die konfiguriert ist, um das Zeitgesetz der Laserstrahlungsmodulation einschließlich Modulationsamplitude, Modulationstiefe, Modulationsfrequenz und Modulationsmodus aus präoperativen Untersuchungsdaten zu bestimmen und um das Gesetz während der Laserbehandlung in Reaktion auf ein Steuersignal von dem Überwachungs-/Diagnose-System (4) zu aktualisieren.

18. Vorrichtung nach Aspruch 2, die konfiguriert ist, um das Bildungsgesetz der räumlichen Laserstrahlungsverteilung aus präoperative Untersuchungsdaten zu bestimmen und um das Gesetz während der Laserbehandlung in Reaktion auf ein Steuersignal von dem Überwachungs-/Diagnose-System (4) zu aktualisieren.

19. Vorrichtung nach Anspruch 2, die konfiguriert ist, um die Parameter der Laserstrahlungsabtastung aus präoperativen Untersuchungsdaten zu bestimmen und um sie während der Laserbehandlung in Reaktion auf ein Steuersignal von dem Überwachungs-/Diagnose-System (4) zu aktualisieren.

20. Vorrichtung nach anspruch 2, die konfiguriert ist, um eine Übereinstimmung zwischen dem Modulationsgesetz und dem Gesetz der räumlichen Bildung der Laserstrahlung anhand der präoperativen Untersuchungsdaten zu schaffen und um die Übereinstimmung während der Laserbehandlung in Reaktion auf ein Steuersignal von dem Überwachungs-/Diagnose-System zu aktualisierten.

21. Vorrichtung nach einem der Ansprüche 1 oder 2, wobei anhand eines opto-elektrischen Signals von dem biologischen Gewebe eine Rückkopplung geschaffen wird.

22. Vorrichtung nach Anspruch 1, die ein chirurgisches Instrument enthält, das betreibbar ist, um den wenigstens einen Sensor für den Zustand des biologischen Gewebes des Überwachungs-/Diagnose-Systems in dem Bereich biologischen Gewebes zu positionierten.

23. Vorrichtung nach Anspruch 22, die konfiguriert ist, um Knorpelgewebe zu behandeln.

## Revendications

1. Dispositif de traitement optique, mécanique est thermique d'un tissu biologique, comportant :
un outil mécanique servant à appliquer une pression locale sur des zones du tissu biologique (8) ;
une source (1) de rayonnement optique dotée d'une unité de régulation de la puissance de rayonnement optique et de la modulation temporelle, le dispositif de traitement de tissu biologique comportant en outre et étant couplé optiquement à un dispositif (2, 3) servant à acheminer un rayonnement optiquement modulé et formé spatialement, selon une loi prédéterminée ;
un système (4) de contrôle-diagnostic assurant la détermination de la répartition spatiale des caractéristiques physico-chimiques et géométriques de la zone de tissu biologique, et assurant une rétroaction sur la base de la répartition spatiale mesurée des caractéristiques physico-chimiques et géométriques de la zone de tissu biologique, ledit système de contrôle-diagnostic étant relié respectivement à la source de rayonnement optique, à l'unité de régulation de a puissance de rayonnement optique et de la modulation temporelle et au dispositif servant à acheminer le rayonnement optique et à former la répartition spatiale de la densité de puissance du rayonnement optique à la surface et ans la masse de la zone de tissu biologique,
ledit système de contrôle-diagnostic comportant au moins un capteur (6) d'état du tissu biologique afin de mesurer des caractéristiques de la zone de tissu biologique dans la région de traitement et à proximité immédiate de celle-ci ;
ledit système de contrôle-diagnostic comportant une unité (7) de traitement de données reliée audit capteur d'état du tissu biologique et configurée pour sélectionner une forme et une taille de la tache de rayonnement et une loi de balayage en fonction de propriétés géométriques et d'une répartition spatiale de contraintes dans la zone de tissu biologique traitée, et pour sélectionner une valeur prédéterminée de la fréquence de modulation du rayonnement optique afin qu'elle corresponde aux fréquences de résonance de vibrations mécaniques induites dans la zone de tissu biologique traitée, et ladite unité de traitement de données étant configurée pour générer, selon un algorithme spécifique, des signaux de commande servant à modifier des paramètres de puissance, de modulation temporelle et des paramètres de la répartition spatiale de la densité de puissance du rayonnement optique sur la base des signaux dudit ou desdits capteurs (6) d'état du tissu biologique.

2. Dispositif selon la revendication 1, la source (1) de rayonnement optique étant une source de rayonnement laser.

3. Dispositif selon la revendication 2, la source de rayonnement laser émettant le rayonnement laser dans une gamme de 0,1 à 11 micromètres.

4. Dispositif selon la revendication 1, le système (4) de contrôle-diagnostic comportant un dispositif (5) de visualisation et d'affichage d'informations et ladite unité (7) de traitement de données étant configurée pour générer des signaux de commande servant à commander les paramètres de rayonnement optique lors du processus d'irradiation.

5. Dispositif selon la revendication 4, le ou les capteurs (6) d'état du tissu biologique du système (4) de contrôle-diagnostic étant configurés pour mesurer la température et la concentration en eau du tissu biologique, les contraintes mécaniques, les caractéristiques de diffusion lumineuse, la vitesse du son dans le tissu biologique, le facteur d'amortissement des ondes opto-acoustiques et des dimensions géométriques du tissu biologique.

6. Dispositif selon la revendication 4, l'unité de traitement de données du système de contrôle-diagnostic réagissant à des signaux reçus en provenance du ou des capteurs d'état du tissu biologique et étant configurée pour délivrer des signaux de commande respectivement à la source de rayonnement optique, à l'unité de régulation de la puissance de rayonnement optique et de la modulation temporelle et au dispositif servant à acheminer le rayonnement optique et à former la répartition spatiale de la densité de puissance du rayonnement optique à la surface et dans la masse du tissu biologique.

7. Dispositif selon la revendication 1, l'unité de régulation de la puissance de rayonnement optique et de la modulation temporelle étant un modulateur électro-optique, un modulateur acousto-optique ou un modulateur mécanique.

8. Dispositif selon la revendication 1, configuré pour moduler le rayonnement optique en modifiant une puissance de pompage de la source de rayonnement laser.

9. Dispositif selon la revendication 1, le dispositif servant à acheminer le rayonnement optique et à former la répartition spatiale de la densité de puissance du rayonnement optique à la surface et dans la masse du tissu biologique comprenant un système optique de formation et un dispositif de balayage électro-optique, couplé optiquement au système optique de formation.

10. Dispositif selon la revendication 1, le dispositif servant à acheminer le rayonnement optique et à former la répartition spatiale de la densité de puissance du rayonnement optique à la surface et dans la masse du tissu biologique comprenant un système optique de formation et un système de trame, couplé optiquement au système optique de formation.

11. Dispositif selon l'une quelconque des revendications 9 ou 10, ledit système optique de formation comportant un segment de fibre optique ou un système lentille-miroir prévu pour acheminer le rayonnement laser de la source de rayonnement optique à la zone de tissu biologique.

12. Dispositif selon la revendication 4, le Dispositif (5) de visualisation et d'affichage d'informations comprenant un endoscope et un afficheur servant à afficher la zone de tissu biologique, ou une tomographie optique cohérente.

13. Dispositif selon la revendication 4, le dispositif (5) de visualisation et d'affichage d'informations étant configuré pour mesurer des caractéristiques géométriques de la zone de tissu biologique.

14. Dispositif selon la revendication 4, une rétroaction étant assurée par le système (4) de contrôle-diagnostic sur la base d'une réponse optique-thermique du tissu biologique au rayonnement laser module temporellement et formé spatialement.

15. Dispositif selon la revendication 2, une rétroaction étant assurée par le système (4) de contrôle-diagnostic sur la base d'une analyse de la répartition spectrale de la réponse du tissu biologique au rayonnement laser modulé.

16. Dispositif selon la revendication 1, une rétroaction étant assurée par le système (4) de contrôle-diagnostic sur la base d'une analyse de l'amplitude et de la phase d'une réponse du tissu biologique au rayonnement laser modulé.

17. Dispositif selon la revendication 2, configuré pour déterminer la loi temporelle de modulation du rayonnement laser, notamment l'amplitude de modulation, la profondeur de modulation, la fréquence de modulation et le mode de modulation, à partir de données d'examen préopératoire, et pour mettre à jour ladite loi au cours du traitement par laser en réaction à un signal de commande provenant du système (4) de contrôle-diagnostic.

18. Dispositif selon la revendication 2, configuré pour déterminer la loi de formation de la répartition spatiale du rayonnement laser à partir de données d'examen préopératoire et pour mettre à jour ladite loi au cours du traitement par laser en réaction à un signal de commande provenant du système (4) de contrôle-diagnostic.

19. Dispositif selon la revendication 2, configuré pour déterminer les paramètres de balayage du rayonnement laser à partir de données d'examen préopératoire et pour les mettre à jour au cours du traitement par laser en réaction à un signal de commande provenant du système (4) de contrôle-diagnostics.

20. Dispositif selon la revendication 2, configuré pour assurer une correspondance entre la loi de modulation et la loi de formation spatiale du rayonnement laser sur la base des données d'examen préopératoire et pour mettre à jour ladite correspondance au cours du traitement par laser en réaction à un signal de commande provenant du système de contrôle-diagnostic.

21. Dispositif selon l'une quelconque des revendications 1 et 2, la rétroaction étant assurée sur la base d'un signal opto-électrique provenant du tissu biologique.

22. Dispositif selon la revendication 1, comportant un instrument chirurgical utilisable pour positionner le ou les capteurs d'état du tissu biologique du système de contrôle-diagnostic dans la zone de tissu biologique.

23. Dispositif selon la revendication 22, configuré pour le traitement d'un tissu cartilagineux.
